# FASCICULE DE BREVET EUROPEEN

(11) **EP 1 521 795 B1**
(45) Date de publication et mention de la délivrance du brevet: **14.03.2012**
(21) Numéro de dépôt: 03760030.1
(22) Date de dépôt: 13.06.2003
(51) Int. Cl.: C08G 65/333, C08G 18/28, C08G 18/80, C08G 18/48

(54) **POLYMERES THERMOSENSIBLES ET GELS THERMOREVERSIBLES OBTENUS A PARTIR DE CES POLYMERES**
HITZEMPFINDLICHE POLYMERE UND THERMOREVERSIBLE GELE DARAUS
HEAT-SENSITIVE POLYMERS AND HEAT REVERSIBLE GELS DERIVED THEREFROM

(30) Priorité: 14.06.2002 FR 0207463
(43) Date de publication de la demande: 13.04.2005
(73) Titulaire: Polymerexpert SA, 33600 Pessac (FR)
(72) Inventeur: PAGNOUX, Anne, F-33114 Le Barp (FR); DOLATKHANI, Marc, F-33610 Cestas (FR); CHAFFAUX, Patricia, F-33400 Pessac (FR)
(74) Mandataire: Ulmann, Catherine Claire
(86) Numéro de dépôt international: PCT/FR2003/001786
(87) Numéro de publication internationale: WO 2003/106536

(56) Documents cités:
- EP-A- 0 963 796
- WO-A-95/26993
- WO-A-99/47072
- GB-A- 2 276 627
- US-A- 4 611 083
- US-A- 5 120 816
- US-A- 5 183 876
- US-A- 5 694 806

## Description

La présente invention concerne un polymère thermosensible apte à former des gels thermoréversibles à haut indice de viscosification ainsi que leur préparation. Elle concerne également des applications de ces gels.

Des domaines d'application envisagés pour ces gels thermoréversibles sont notamment, mais non exclusivement, des compositions thérapeutiques ou non thérapeutiques, en particulier cosmétiques, pour le traitement du corps humain ou animal.

Les compositions à gélification réversible sont définies comme des solutions dont la variation de viscosité est liée à une modification des conditions environnementales. Lorsque cette modification de viscosité intervient lors d'un changement de température, on parlera de gels thermoréversibles et les polymères constitutifs de la formulation sont identifiés comme des "polymères thermo-gélifiants" ou encore des "polymères thermosensibles". Ces polymères sont formés de parties hydrophobes, thermosensibles, et de parties hydrophiles. La formation de gel est expliquée par l'auto-association des portions thermosensibles en micro-domaines hydrophobes ; l'ensemble du polymère étant maintenu en solution par les segments hydrophiles. Les propriétés de viscosification et celles du gel sont alors contrôlées par la longueur respective des différents segments et par le ratio hydrophobe/hydrophile (L.E. Bromberg, Adv. Drug Delivery Reviews 31 (1998) 197-221).

On connaît des polymères thermosensibles destinés à former des gels thermoréversibles en solution aqueuse, la viscosité desdits gels évoluant de façon réversible en fonction de leur température. Ces polymères thermosensibles présentent des portions hydrophobes susceptibles de s'agréger ensemble pour former des micelles lorsque la température du milieu est élevée pour atteindre celle de leur température critique de solution ; les portions hydrophiles reliant entre elles lesdites micelles. De la sorte, une augmentation de la température du milieu aqueux dans lequel sont dissous ces polymères thermosensibles est susceptible de le transformer d'un état liquide à un état visqueux formant gel.

De tels polymères sont bien connus sous le nom générique de poloxamère. Ce sont des copolymères à blocs d'oxyde de propylène et d'oxyde d'éthylène, ou polyoxyalkylènes, susceptibles d'être synthétisés notamment selon les procédés décrits dans les brevets US 4 188 373 et US 4 478 822. Les polymères thermosensibles ainsi obtenus permettent de formuler des compositions aqueuses présentent des températures critiques de solution comprises entre 24 et 40°C. Cependant, de telles formulations contiennent nécessairement de 15 à 50% de polymères thermosensibles pour obtenir une variation significative de la viscosité de sorte, qu'elles sont initialement extrêmement visqueuses.

En outre, malgré le pourcentage élevé de polymères thermosensibles qu'elles contiennent, ces formulations ne présentent que des variations de viscosité inférieures à une décade à leur température critique de solution.

Plus récemment, des travaux ont été menés pour obtenir des compositions alliant des propriétés de thermogélification et de bioadhésivité. Dans cette optique, plusieurs brevets ont revendiqué des formulations à gélification réversible élaborées par simples mélanges physiques d'un polymère thermo-gélifiant (poloxamère) et d'un polymère sensible au pH, choisi parmi les polyacides carboxyliques (Carbopol) (US Patent N° 5,252,318, FR 2 802 097 et EP 0551 626). A la température critique de solution (LCST - Lower Critical Solution Temperature), la variation de viscosité observée est d'environ 5 à 8 fois la viscosité initiale mais la concentration minimale requise reste élevée : au moins 12% massique de polymères. Les polyacides acryliques ont majoritairement été employés pour leur propriété d'adhésivité. Dans les dernières innovations, des segments de polyacides acryliques ont été associés chimiquement à des segments de poloxamères. Outre le caractère biadhésif et "pH sensible", la partie polyacide acrylique confère au matériau une plus grande solubilité dans l'eau. La présence du segment hygrophile favorise la solubilisation du copolymère et limite ainsi la séparation de phase. Il apparaît que les copolymères alternés de monomères thermo- et pH sensibles perdent rapidement leur propriété thermo-gélifiante lorsque le taux de monomère sensible au pH augmente ; les copolymères à blocs sont préférés.

La demande internationale WO95/24430 décrit des copolymères greffés ou à blocs de polyacides acryliques (PAA) et de polymères thermosensibles. La composante thermosensible du matériau est assurée par des polymères Pluronics® ou du poly(isopropylacrylamide) (NIPAm). Selon le taux d'ionisation des fonctions carboxyliques, la stabilité des gels et la valeur de la température critique de gélification sont légèrement différentes.

La copolymérisation s'effectue soit par réaction de condensation des fonctions acides du PAA avec l'extrémité réactive modifiée du Pluronic (monoamination des extrémités hydroxy-) - le copolymère Pluronic-g-poly(acide acrylique) présente des greffons thermosensibles - , soit par réaction de condensation entre le polyacide acrylique et le poly(isopropylacrylamide), tous deux modifiés à une extrémité par des fonctions inter-condensables (amine et acide) - le copolymère Pluronic-b-poly(NIPAm) est formé par deux blocs liés chimiquement.

Comparativement aux mélanges physiques de poloxamères et de polyacides, la gélification thermoréversible des copolymères d'Hoffman (WO95/24430) est obtenue avec des compositions de plus faibles concentrations en polymère : les formulations contenant de 1 à 3% massique en copolymère présentent une plage de température critique de gélification bien définie, entre 20°C et 40°C, pour une gamme de pH allant de 4 à 8. Un état de la technique antérieure est également décrit dans WO99/47072 et WO95/26993

Cependant, la variation de viscosité pour ces compositions n'atteint pas une décade et une séparation de phase en micro-domaines est observée à la température critique de gélification, ce qui se traduit par une opacification du milieu. De plus, les synthèses sont réalisées en plusieurs étapes : modification contrôlée des fonctions terminales des polymères employés, condensation ou copolymérisation en chaîne et enfin séparation/purification des produits désirés.

Suite à ces travaux, Bromberg *et al.* ont décrit des copolymères en peigne de polyacide acrylique et de poloxamère et leur nouvelle voie d'obtention (WO 9700275). Dans une première étape, des radicaux sont créés sur la chaîne de poloxamère par arrachement d'hydrogène sur le segment de poly(oxyde de propylène). La polymérisation radicalaire en chaîne de l'acide acrylique est alors amorcée à partir des macroradicaux poloxamère.

Le système obtenu, nommé Smart Hydrogels™, présente une parfaite clarté avant et au point de gélification ; la transition sol-gel de solutions aqueuses faiblement concentrées en copolymères (1 à 5 % massique) se produit dans un intervalle étroit de température (10°C), entre 25 et 40°C et se traduit par une augmentation de viscosité d'environ au moins 30 fois la viscosité initiale. Le gel ainsi formé se comporte comme un solide viscoélastique et conserve sa viscosité quelle que soit la vitesse de cisaillement appliquée.

Deux inconvénients concernant ce système ont été relevés par les inventeurs de la présente invention : la bioadhésivité de l'hydrogel est limitée par une mauvaise accessibilité des parties poly(acide acrylique) et les compositions ont une stabilité réduite en raison de l'oxydation initiale du polymère Pluronic® pour créer le radical amorçant.

Pour améliorer ces propriétés, Bromberg *et al*. ont élaboré de nouveaux copolymères linéaires à blocs en gardant le poloxamère et le poly(acide acrylique), respectivement comme composés thermosensible et hydrophile. L'originalité de ces copolymères est qu'ils sont composés d'un bloc central de poloxamère modifié à ses deux extrémités par des blocs polyacides. Pour leur obtention, les deux extrémités du poloxamère sont préalablement fonctionnalisées par des groupes acryliques ou thiol permettant l'amorçage de la polymérisation radicalaire de l'acide acrylique. Ces triblocs montrent une gélification réversible à température du corps (25-40°C), à des pH compris entre 3 et 13. Les solutions faiblement concentrées (1 à 4% massique) subissent alors une augmentation de viscosité pouvant aller jusqu'à 2 décades.

Mais là encore, la voie de synthèse choisie est multiétapes et il est nécessaire d'éliminer, en cours ou en fins de fabrication, les monomères résiduels par des traitements importants (extraction au sohxlet, dialyse, multiples précipitations...).

Ainsi, plusieurs systèmes à gélification réversibles sont connus à ce jour. Toutefois, ils nécessitent un taux élevé de solide et/ou conduisent à un gain faible en viscosité des formulations à la LCST. Enfin, les systèmes les plus récents sont obtenus à partir de poloxamères à extrémités modifiées, ce qui nécessite des synthèses multiétapes et des opérations de séparations/purifications peu compatibles avec un procédé de production industriel.

La présente invention fournit des polymères thermosensibles qui permettent d'obtenir non seulement des gels physiques thermoréversibles avec une faible concentration en polymère, mais aussi des gels physiques thermoréversibles à haut indice de viscosification, dont la viscosité augmente fortement à leur température critique de solution.

Par gel physique, on entend un gel résultant de l'association des chaînes polymères grâce à la formation de liaisons non covalentes, de type ioniques, dipolaires, liaisons hydrogène ou interactions hydrophobes entre les chaînes.

En outre, un but de la présente invention est également de fournir des polymères thermosensibles permettant d'obtenir des gels thermoréversibles à des températures sensiblement égales aux températures corporelles pour formuler des compositions cosmétiques et pharmaceutiques efficaces.

Plus précisément, l'invention concerne des polymères comprenant des chaînes polymères de type terpolymère constituées de polyoxyde d'éthylène (POE) et de polyoxyde de propylène (PPO) de la forme POE-PPO-POE modifiées en leurs extrémités par des groupements qui peuvent être essentiellement d'autres chaînes de POE-PPO-POE, des segments acides, des groupements amines ou des POE, ces chaînes étant reliées aux chaînes de terpolymères par des ponts chimiques qui sont constitués par des ponts uréthane, des ponts urée, des ponts allophanate et des ponts biuret. Tous ces ponts peuvent être présents soit dans une même chaîne de polymères, soit dans des chaînes différentes et, comme cela ressort de l'exposé qui suit, la proportion de ces différentes liaisons dépend essentiellement des conditions opératoires. En effet, comme cela ressort de l'exposé qui suit, en fonction des proportions des différents réactifs, on accède soit à des polymères de type linéaire dans lesquels les chaînes polyoxyalkylène de type terpolymère sont allongées essentiellement par l'intermédiaire de groupements chimiques qui leur sont reliés par des ponts de type carbamate et urée, soit à des polymères ramifiés présentant en outre des groupements de type allophanate et/ou biuret.

Or il est apparu aux inventeurs de la présente invention que, la présence dans ces polymères de liaisons de type urée et éventuellement de type biuret améliorait grandement les propriétés du gel formé à partir de ces polymères, du fait de la création de liaisons hydrogène additionnelles entre les différentes chaînes polymères.

Selon un premier objet, la présente invention propose un polymère thermosensible hydrosoluble apte à former des gels physiques thermoréversibles à haut indice de viscosification, caractérisé en ce qu'il comprend à la fois des enchaînements comprenant au moins une chaîne linéaire de type polyoxyalkylène triblocs thermosensible constituée de blocs de polyoxyde d'éthylène (POE) et de blocs de polyoxyde de propylène (PPO), ladite chaîne étant de la forme POE-PPO-POE et étant allongée à au moins une de ses extrémités par un groupement organique via une liaison carbamate et des enchaînements comprenant au moins une chaîne linéaire de type polyoxyalkylène triblocs POE-PPO-POE allongée à au moins une de ses extrémités par un groupement organique via une liaison urée.

Ainsi, une caractéristique du polymère thermosensible selon l'invention, réside dans l'allongement de la chaîne linéaire des polyoxyalkylènes thermosensibles et dans l'introduction de groupements uréiques dans la chaîne polymère, de façon à leur conférer un plus haut poids moléculaire et à apporter de nouvelles fonctions aptes à induire des interactions supplémentaires de type liaisons hydrogène. L'allongement est alors réalisé en reliant les groupements organiques aux chaînes linéaires par des liaisons carbamate et urée. De la sorte, grâce à des polymères thermosensibles de plus haut poids moléculaire, contenant des ponts urée, la viscosité des gels qu'ils permettent de formuler, à leur température critique de solution, est accrue.

Avantageusement, la structure dudit polymère thermosensible comporte au moins une chaîne linéaire de type polyoxyalkylène thermosensible constitué de trois blocs (polyoxyde d'éthylène-polyoxyde de propylène-polyoxyde d'éthylène) allongée à chacune de ses deux extrémités par un groupement organique via une liaison carbamate ou urée. De la sorte, le poids moléculaire du polymère est encore accru et de nouveaux groupements aptes à former des interactions de type liaisons hydrogène sont introduits dans la chaîne polymère, ce qui confère une encore plus grande viscosité aux gels formulés, à la température critique, sans que la viscosité du gel soit importante en dehors de cette température critique de solution.

Selon un mode de mise en oeuvre de l'invention particulièrement avantageux, lesdites chaînes linéaires de type polyoxyalkylène triblocs thermosensible répondent à la formule : 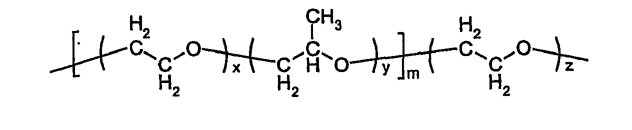 dans laquelle, 20< x <120, 20< y <120, 20< z <120, et m>0. Ainsi, la chaîne polyoxyalkylène présente un bloc d'oxyde de propylène linéaire dont chaque extrémité est reliée à un bloc d'oxyde d'éthylène. De façon préférentielle, la chaîne linéaire polyoxyalkylène est symétrique, m étant égal à 1 et x étant sensiblement égal à z. Ce sont les extrémités de la chaîne qui sont reliées au groupement organique par des liaisons carbamate et/ou urée.

De façon particulièrement avantageuse, lesdits groupements organiques renferment des radicaux susceptibles d'être reliés aux chaînes polyoxyalkylène par une liaison carbamate ou urée et sont choisis parmi :

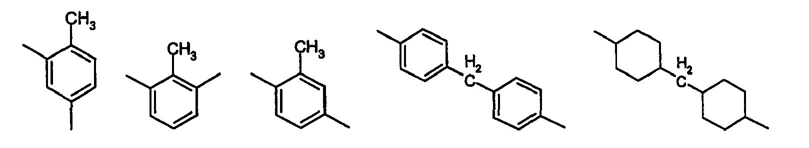

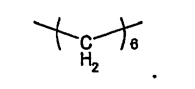

Ainsi, selon cette caractéristique de l'invention, les groupements organiques renferment au moins un desdits radicaux, notamment à l'une de ses extrémités.

Le radical est relié, d'une part à la chaîne polyoxyalkylène par une liaison carbamate ou urée, et d'autre part à une autre molécule, également par un groupement carbamate ou urée.

Selon une variante de réalisation de l'invention, les groupements organiques renferment au moins un desdits radicaux et des motifs acide reliés entre eux par des liaisons carbamate ou urée. De la sorte, le motif acide est espacé de la chaîne polyoxyalkylène par un desdits radicaux, lequel est relié à la chaîne polyoxyalkylène et au motif acide par deux liaisons carbamates ou urée distinctes. On comprend en outre que le groupement organique est susceptible d'être constitué alternativement par lesdits radicaux et par les motifs acide et qu'il est susceptible de s'étendre à chacune des extrémités de ladite chaîne polyoxyalkylène ou qu'il est susceptible de rassembler entre elles deux des dites chaînes de poloxyalkylène. De la sorte, grâce aux fonctions acide dudit motif, le polymère thermosensible, objet de l'invention, est susceptible de voir ses propriétés rhéologiques, non seulement variées en fonction de la température mais aussi en fonction du pH du milieu dans lequel il se trouve. De plus, la présence de groupement acide confère au polymère thermosensible la propriété de bioadhésivité.

Selon une autre variante avantageuse, ledit groupement organique renferme lesdits radicaux et des motifs amine tertiaire reliés entre eux par des liaisons carbamate ou urée. Ainsi, les fonctions amine qui sont susceptibles de capter un proton, permettent de faire varier les propriétés du polymère en fonction de l'acidité du milieu.

En outre, préférentiellement, le groupement organique renferme alternativement au moins une séquence : radical, motif amine, radical et motif acide, les éléments de cette séquence étant reliés deux à deux par une liaison carbamate ou urée

Selon un autre mode particulier de mise en oeuvre de l'invention, ledit groupement organique renferme une chaîne de type polyoxyde d'éthylène. Cette chaîne polyoxyde d'éthylène peut être espacée de la chaîne polyoxyalkylène thermosensible par un desdits radicaux et reliée à ce dernier par une liaison carbamate ou urée. En outre, la chaîne polyoxyde d'éthylène qui présente avantageusement un poids moléculaire inférieur à 1000 est susceptible d'être associée à des motifs acide ou amine dans le groupement organique.

De façon particulièrement avantageuse, ledit groupement organique présente des ramifications constituées par des liaisons allophanate ou biuret. Ces liaisons, formées, comme on l'expliquera plus en détail dans la description du procédé de synthèse, par la réaction d'une fonction isocyanate sur une liaison carbamate ou urée, engendrent des ramifications grâce à l'azote tri-substitué de la liaison carbamate ou urée. Ainsi, les radicaux sont susceptibles d'être reliés entre eux grâce aux liaisons allophanate ou biuret. En outre, de façon particulièrement avantageuse, le polymère thermosensible amélioré comprend une pluralité de chaînes linéaires de type polyoxyalkylène triblocs thermosensible reliées entre elles par un ou des groupements organiques par l'intermédiaire de liaisons carbamate ou urée, soit linéairement, les chaînes linéaires polyoxyalkylène étant espacées les unes des autres par des groupements organiques, soit de manière ramifiées grâce aux liaisons allophanate ou biuret.

De plus, on comprend que les groupements organiques peuvent renfermer des motifs acide, amine ou des chaînes polyoxyéthylène.

Selon un deuxième objet, la présente invention propose un procédé de synthèse d'un polymère thermosensible amélioré apte à former des gels physiques thermoréversibles à haut indice de viscosification et, tout particulièrement des polymères définis précédemment, lequel procédé comprend la réaction d'au moins un polymère P linéaire de type polyoxyalkylène triblocs thermosensible présentant au moins une fonction hydroxy terminale avec au moins une molécule organique porteuse d'au moins une fonction isocyanate de façon à les relier ensemble par des liaisons carbamate ou urée.

Cette synthèse s'effectue de façon particulièrement simple en milieu solvant en une seule étape et sans purification intermédiaire des polymères P qui contiennent généralement au moins des traces d'eau dont la présence permet d'introduire au cours de la réaction des liaisons de type urée dans les chaînes formées.

Comme cela ressort de l'exposé qui suit, en vue d'augmenter la proportion de liaisons urée, il est possible d'ajouter de l'eau dans le milieu réactionnel à raison d'une quantité totale d'eau de préférence de 0,1% à 0,6% massique par rapport au terpolymère, de préférence encore de 0,3 à 0,6%.

Ainsi, une caractéristique de l'invention est d'allonger un polymère P linéaire de type polyoxyalkylène triblocs thermosensible présentant au moins une fonction hydroxy terminale en faisant réagir des fonctions isocyanate sur les fonctions hydroxy du polymère P en présence d'eau.

De façon avantageuse, ledit polymère P présente au moins deux fonctions hydroxy terminales de manière à pouvoir être allongé à chacune de ses extrémités.

Selon un mode de mise en oeuvre particulièrement avantageux de l'invention, ledit polymère P a pour formule générique : 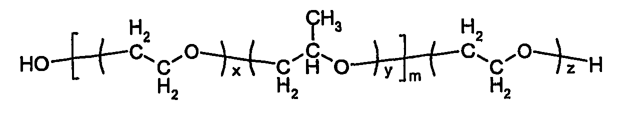 dans laquelle, 20< x <120, 20< y <120, 20< z <120, et m>0. Préférentiellement, m est égal à 1 et x égal z.

De façon avantageuse, la molécule organique comprend deux fonctions isocyanate, de sorte qu'une seule molécule organique peut relier entre elles deux molécules comprenant des groupements hydroxy via deux liaisons carbamate ou urée obtenues par réaction de condensation des fonctions isocyanate et des fonctions hydroxy, en présence d'eau.

Selon un mode préféré de mise en oeuvre de l'invention, la molécule organique est choisie parmi :

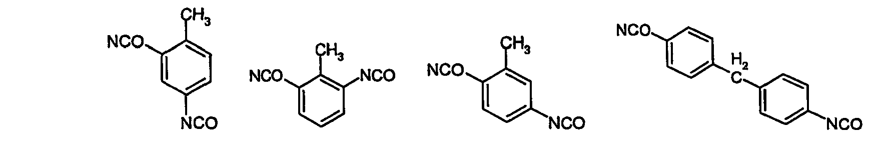

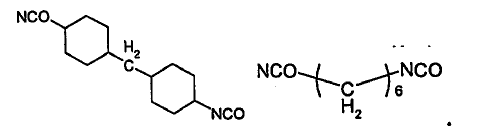

Le choix de telle ou telle molécule organique permet d'obtenir une plus ou moins grande vitesse de réaction de condensation grâce au choix d'une molécule organique présentant au moins un cycle benzénique ou non et grâce à la position de la fonction isocyanate sur le cycle. De la sorte, différents polymères thermosensibles sont susceptibles d'être obtenus.

Selon un autre mode préféré de mise en oeuvre de l'invention, ladite réaction est mise en oeuvre en présence d'au moins une autre molécule organique porteuse d'au moins une fonction hydroxy, avantageusement deux, en présence d'eau. Ainsi, grâce aux deux fonctions isocyanate des molécules organiques, ladite autre molécule organique est susceptible d'être reliée à la molécule organique par une liaison carbamate ou urée, qui elle-même est reliée à ladite chaîne dudit polymère P linéaire de type polyoxyalkylène thermosensible. De plus, lorsque ladite autre molécule présente deux fonctions hydroxy, on comprend que l'on peut allonger ledit polymère thermosensible à chacune de ses extrémités, alternativement, par une molécule organique présentant deux fonctions isocyanate et une autre molécule, les molécules organiques étant reliées aux autres molécules organiques par des liaisons carbamate ou urée.

Dans un mode particulier de mise en oeuvre de l'invention, selon ledit autre mode préféré et de façon préférentielle, ladite autre molécule organique comprend en outre au moins une fonction acide carboxylique, avantageusement deux. De la sorte, il est aisé de produire des polymères thermosensibles améliorés sensibles aux variations de pH et dont la viscosité du gel qu'il permet de réaliser évolue non seulement avec la température, mais aussi avec l'acidité du milieu.

Avantageusement, ladite autre molécule a pour formule :

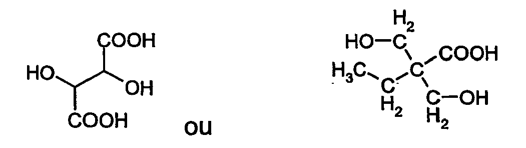

De façon parallèle audit mode particulier de mise en oeuvre de l'invention précédent, ladite autre molécule organique comprend, avantageusement, au moins une fonction amine tertiaire procurant également aux gels formulés avec le polymère thermosensible amélioré, une sensibilité aux variations de pH. Avantageusement, ladite autre molécule a pour formule :

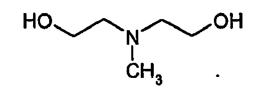

Selon ledit autre mode préféré de mise en oeuvre de l'invention et avantageusement, ladite autre molécule est un polyoxyde d'éthylène monohydroxylé susceptible d'augmenter la stabilité et les propriétés de viscosification du gel contenant ledit polymère thermosensible amélioré.

Selon un troisième objet, la présente invention propose un polymère thermosensible amélioré présentant en solution une faible viscosité à température ambiante et apte à former des gels physiques thermoréversibles à haut indice de viscosification pour des températures supérieures à 25°C. Sa structure comprend au moins une chaîne linéaire de type polyoxyalkylène triblocs thermosensible allongée à l'une de ses extrémités au moins par un groupement organique via une liaison carbamate ou urée. Elle est susceptible d'être obtenue par un procédé de synthèse comprenant la réaction d'au moins un polymère P linéaire de type polyoxyalkylène triblocs thermosensible présentant au moins une fonction hydroxy terminale avec au moins une molécule organique porteuse d'au moins une fonction isocyanate, en présence d'eau, de façon à les relier ensemble par ladite liaison carbamate ou urée.

Selon un quatrième objet, la présente invention propose un gel thermoréversible, comprenant au moins un polymère thermosensible conforme au premier objet ou au moins un polymère thermosensible obtenu selon un procédé de synthèse conforme au deuxième objet.

Le gel formé est un gel physique.

Le gel thermoréversible de l'invention contient de 1 à 10% en poids dudit polymère thermosensible amélioré et plus avantageusement de 1 à 5% dudit polymère thermosensible amélioré obtenu en présence d'eau et contenant ainsi des groupements urée.

Selon un cinquième objet, la présente invention propose une composition pharmaceutique ou non pharmaceutique, en particulier une composition cosmétique, destinée au traitement ou au soin du corps humain comprenant un produit en solution de faible viscosité à température et apte à former un gel physique thermoréversible de haute viscosité à des températures supérieures à 25°C selon ledit quatrième objet.

Selon un sixième objet, l'invention concerne un élément prothétique susceptible d'être inséré dans un organe du corps humain, caractérisé en ce qu'il comprend un gel thermoréversible selon ledit quatrième objet.

D'autres particularités et avantages de l'invention ressortiront à la lecture de la description détaillée faite ci-après de modes de réalisation particuliers de l'invention, données à titre indicatif mais non limitatif, ainsi que des exemples et des figures.
- La Figure 1 illustre la courbe de viscosité d'un gel obtenu selon l'exemple 1.
- La Figure 2 illustre les courbes de viscosité d'un gel obtenu selon l'exemple 3.
- La figure 3 présente la courbe de viscosité d'un gel obtenu selon l'exemple 4.
- La figure 4 présente la courbe de viscosité d'un gel obtenu selon l'exemple 5.

L'invention concerne des copolymères hydrosolubles thermosensibles améliorés, linéaires ou ramifiés qui présentent en solution une faible viscosité à température ambiante et qui sont aptes à former des gels thermoréversibles à haut indice de viscosification à des températures supérieures à 26°C et un procédé de synthèse de tels polymères. En outre, elle concerne également l'application d'un tel gel thermoréversible.

Selon l'invention, on synthétise des polymères hydrosolubles thermosensibles améliorés, permettant d'obtenir des gels thermoréversibles dont la viscosité augmente d'au moins un facteur 1000 lorsque la température dépasse 25°C pour des concentrations en polymères thermosensibles dans l'eau inférieures à 10%. La synthèse des polymères thermosensibles améliorés est effectuée à partir de copolymères thermosensibles bien connus : les polyoxyalkylènes ou poloxamères, à fonctions hydroxy, terminal. Les copolymères choisis sont des triblocs et ont pour formule générique : 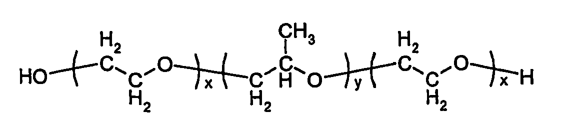 dans laquelle, 20< x <120 et 20< y <120. Ces polymères thermosensibles, commercialisés par exemple sous le non de Pluronics, renferment 25% en mole de motif oxyde de propylène et permettent une augmentation de la viscosité d'uniquement un facteur 10 lorsque la température passe de 20°C à 30°C pour des concentrations supérieures à 15% dans l'eau.

Bien évidemment, un mélange de ces types de polymères thermosensibles est susceptible d'être utilisé pour réaliser des polymères thermosensibles améliorés.

Le concept inventif de l'invention réside notamment dans le couplage des polymères thermosensibles bien connus avec des groupements organiques dont la taille est inférieure à celle desdits polymères. Ces couplages sont susceptibles d'être obtenus par réaction de composés organiques de type di-isocyanate sur les fonctions hydroxy du polymère thermosensible en présence d'eau pour former des liaisons uréthane et/ou urée ainsi que des groupements allophanate et/ou biuret. De plus, les procédés à mettre en oeuvre sont simplifiés, car on peut effectuer les synthèses dans un même réacteur, les di-isocyanate réagissant par polycondensation sur les fonctions hydroxy des polymères thermosensibles, sans étape d'élimination des traces d'eau habituellement présentes dans les copolymères de type polyoxyalkylène.

Ainsi, dans un réacteur équipé d'une agitation mécanique, d'un réfrigérant et d'une arrivée d'azote, on dissout le polymère triblocs POE-PPO-POE dans un solvant, de préférence dans la butanone à une température de l'ordre de 70°C. Le di-isocyanate est introduit goutte-à-goutte. A la fin de l'ajout, le catalyseur est additionné. La réaction se poursuit à 70°C sous agitation jusqu'à complète disparition des isocyanates.

L'avantage de la synthèse est qu'elle est réalisée, en une seule étape, dans un seul réacteur. Le second avantage de cette synthèse est qu'elle est réalisée à partir des constituants commerciaux, sans aucun traitement de purification supplémentaire. Ainsi, le copolymère triblocs POE-PPO-POE est utilisé sans être séché. Il contient alors généralement 0,3%+/- 0,05% en masse d'eau et cette eau conduit lors de la synthèse à la formation de ponts urée. C'est dans ce cas précis que les polymères thermogélifiants obtenus sont les plus efficaces car il y a, outre les ponts uréthane et allophanate, des ponts urée et biuret qui peuvent donner lieu à des interactions de type liaisons hydrogène qui s'additionnent aux interactions hydrophobes des PPO.

On note que la quantité d'eau acceptable pour former des polymères thermogélifiants est avantageusement de 0,1 à 0,6% en masse par rapport au terpolymère. A 0,6% d'eau, la viscosité de la solution de notre polymère est légèrement supérieure à celle des autres solutions de polymère amélioré mais la viscosité du gel correspondant est également supérieure à celle des autres gels. Il y a donc un effet bénéfique de l'eau (et par là même des groupes urée) sur l'aptitude des polymères à faire gélifier à des températures supérieures à 25°C des solutions aqueuses.

Les polymères les plus efficaces (les plus aptes à faire gélifier l'eau) sont ceux obtenus avec 0,3 à 0,6% d'eau, introduite dès le début de la synthèse dans le milieu réactionnel par l'intermédiaire des constituants et/ou par ajout d'eau.

Ces polymères peuvent être alors utilisés seuls de préférence à raison de 3,5 à 5% massique dans les solutions aqueuses.

En association avec des composés tels des polyacides réticulés de type CARBOPOL, le polymère amélioré peut-être utilisé à moins de 3% et préférentiellement entre 1% et 2,5%. Cet effet de synergie est obtenu avec 0,05% à 1 % de polyacide réticulé.

Dans le cas de polymères élaborés dans des conditions anhydres (séchage du POE-PPO-POE), la quantité de polymère à utiliser serait d'au moins 10% massique pour atteindre le même gain en viscosité.

Les polymères les plus efficaces sont obtenus avec 0,3% d'eau en masse par rapport à la masse de polymères triblocs et avec des fonctions acide ou POE sur la chaîne, avec un rapport NCO/OH=1,1/1 (en comptant l'apport de l'eau contenu dans le triblocs ou 2/1 sans compter l'eau).

Les propriétés d'augmentation de viscosité avec la température peuvent être ajustées en fonction de la concentration en eau dans le milieu réactionnel et donc en fonction de la proportion de groupements urée, biuret, allophanate et uréthane.

Les di-isocyanate susceptibles d'être utilisés pour la synthèse des polymères thermosensibles améliorés sont notamment les composés suivant : 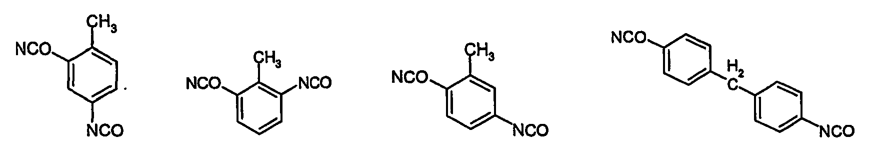 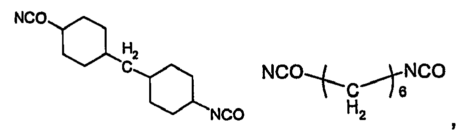 lesquels sont choisis en fonction de la cinétique de réaction et par conséquent du polymère final que l'on souhaite obtenir.

En outre, comme on l'expliquera plus en détail dans la suite de la description, ils permettent d'insérer dans les groupements organiques, reliant les chaînes de polyoxyalkylène d'autres fonctionnalités permettant, notamment, de rendre le gel formulé avec le polymère thermosensible amélioré, sensible au pH du milieu.

Toutefois, comme on l'expliquera dans la suite de la description, un des avantages important des di-isocyanate est de pouvoir introduire des ramifications dans les groupements organiques reliant les polymères thermosensibles et également des groupements urée susceptibles de former par liaison hydrogène des intéractions supplémentaires entre les chaînes de polymères thermosensibles.

Comme exposé précédemment, les polymères de l'invention sont soit linéaires soit ramifiés.

On décrira tout d'abord la structure de polymères thermosensibles améliorés linéaires de façon générale et on donne ensuite des exemples particuliers de procédés de synthèse. Ensuite, on décrira des polymères thermosensibles améliorés ramifiés avec dans la partie exemple des exemples particuliers.

Un premier type de polymère thermosensible amélioré présente la formule générale :
P-(L-P)ₘ, ou L-(P-L)ₘ, dans lesquelles 1 ≤ m ≤ 5.

Le symbole P représente le polymère thermosensible et il est de la forme : 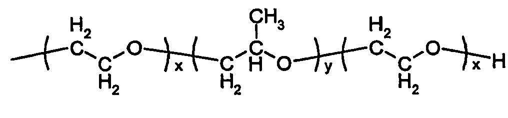 s'il se situe en bout de chaîne ou de la forme : 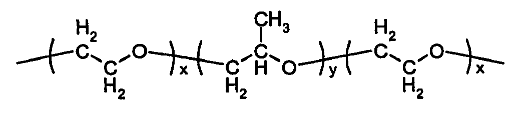 s'il se situe dans la chaîne du polymère.

Le symbole L, correspondant au groupement organique, est dans ce premier type de polymère, choisi parmi les :

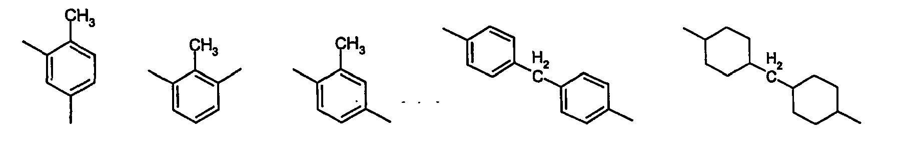

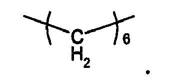

Le groupement L et les polymères thermosensibles P sont reliés entre eux par des fonctions carbamate, et plus particulièrement uréthane, de forme générale : 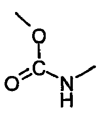 et/ou par des fonctions urée de forme générale -NH-CO-NH. A l'extrémité de la chaîne, le groupement L est susceptible d'être terminé par un groupement azoté.

Un premier exemple de synthèse de polymère thermosensible amélioré correspondant au premier type de polymère décrit ci-dessus est donné dans l'exemple 1. Les proportions des différents constituants et les conditions de mise en oeuvre sont données pour des synthèses de laboratoire, mais elles sont susceptibles d'être adaptées à des conditions industrielles en augmentant les proportions et en adaptant la mise en oeuvre. Ce sera le cas pour tous les exemples de synthèse décrits ci-après.

Un deuxième exemple de synthèse de polymère thermosensible amélioré correspondant à ce premier type de polymère est décrit dans l'exemple 2.

Un deuxième type de polymère thermosensible amélioré, selon l'invention, présente la formule générale : (A-L)ᵣ₁-(P-L)ₘ,-(A-L)ᵣ₂,
ou, (A-L)ᵣ₁-[(P-L)ₘ,-(A-L)ᵣ₂]ₙ-(P-L)ₘ,-(A-L), dans laquelle m', r1, r2 ≥1. P et L correspondent aux structures précédemment décrites. De préférence, r1 et r2 sont compris indépendamment l'un de l'autre entre 1 et 1000.

Le symbole A, quant à lui, correspond soit à des blocs acide soit à des blocs amine tertiaire ou encore à des polyoxyde d'éthylène. Ce deuxième type de polymère peut également renfermer deux de ces différents blocs ou les trois.

On comprend que le groupement organique reliant les polymères thermosensibles est susceptible d'être constitué par un enchaînement de molécule de type L et A reliées les unes aux autres par des fonctions carbamate. Un avantage de ces groupements organiques est qu'ils sont ionisables et que la viscosité du gel formulé avec ce deuxième type de polymère varie avec le pH du milieu.

Les exemples 3, et 4 ci-dessous correspondent à des exemples de synthèse de polymères thermosensibles améliorés correspondant à ce deuxième type de polymère.

Un troisième type de polymère thermosensible amélioré présentant des ramifications est susceptible d'être obtenu conformément à l'invention. Ces ramifications sont formées par réaction de fonctions isocyanate sur des fonctions carbamate et/ou urée pour former des liaisons allophanate ayant la structure suivante : 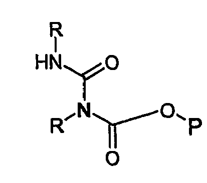 et/ou biuret
où l'azote N de la fonction isocyanate ayant réagi initialement sur la fonction hydroxy du polyoxyalkylène est relié au carbone hybridé sp² d'une seconde fonction isocyanate.

Selon ce troisième type de polymère dont des exemples sont donnés dans les exemples 5, 6, 7 et 8 une forme générale peut être donnée par la formule suivante : 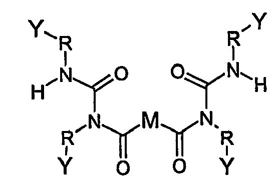 dans laquelle M est susceptible de correspondre au premier ou au deuxième type de polymères thermosensibles améliorés tel que décrit dans les exemples correspondants aux deux types de polymères précédents. R correspond au radical des di-isocyate précédemment décrit et Y peut correspondre à une fonction terminale éthyle ou méthyle, à un groupe amino ou encore à un autre polymère thermosensible amélioré.

Ainsi, on comprend que non seulement ces polymères sont susceptibles d'être ramifiés grâce aux liaisons allophanate ou biuret, mais aussi qu'ils peuvent comprendre une pluralité de polyoxyalkylène reliés entre eux par des groupements organiques susceptibles de comporter des di-isocyanate seuls ou des di-isocyanate couplés avec des blocs acide, basique ou autres.

Les polymères thermosensibles améliorés faisant l'objet de la présente invention présentent le double avantage de former des gels thermoréversibles présentant une forte augmentation de leur viscosité pour des concentrations relativement faibles, avantageusement de l'ordre de 5%. En outre, grâce aux différentes possibilités de synthèse, les plages de température dans lesquelles la viscosité augmente sont susceptibles d'être ajustées et elles peuvent être modulées en fonction du pH.

Les gels sont obtenus à partir d'une solution contenant de 1 à 10% du polymère selon l'invention, de préférence de 1 à 5%, de préférence de 3,5 à 5%.

La solution aqueuse du polymère peut être obtenue de deux façons : à partir du polymère précipité ou grâce à un échange de solvant en fin de synthèse du dit polymère.

Dans le premier cas, le polymère précipité et séché est ajouté dans de l'eau à un, pH de préférence de l'ordre de 7 et la dissolution est réalisée à température proche de l'ambiante (15-23°C) durant 12 à 24 heures. Des agents antimoussants peuvent-être ajoutés pour limiter la formation de mousse et accélérer ainsi la dissolution.

Dans le second cas, en fin de synthèse, lorsque toutes les fonctions isocyanate ont été consommées, de l'eau est ajoutée dans le milieu réactionnel et le solvant organique de la réaction est éliminé sous vide. La quantité d'eau à pH 7 initialement introduite est calculée de façon à obtenir après évaporation du solvant de la réaction un extrait sec de 3,5 à 5%. Cette opération de transfert de solvant permet de s'affranchir de la dissolution lente du polymère.

Les solutions aqueuses de polymères thermogélifiant amélioré ainsi préparées sont liquides à température ambiante [viscosité dynamique entre 35 et 100cP (entre 35.10⁻³ et 0.1 Pa.s)] et se gélifient lorsque leur température se situe entre 25°C et 40°C. La gélification de la solution se traduit par une augmentation de la viscosité d'au moins 3 décades (mesure réalisée sous un cisaillement de 0,3 s⁻¹) et la formation d'une masse qui ne peut plus s'écouler. Notons que le gel formé peut-être rapidement déstructuré si on le soumet à un cisaillement important : il est rhéofluidifiant. Cette propriété est un avantage notamment pour les applications cosmétiques dans lesquelles la solution aqueuse thermogélifiante est diffusée sous forme de spray. En effet, lors de cette opération, la solution de polymère thermosensible amélioré est fortement cisaillée et donc très liquide, puis au contact de la peau la solution se gélifie.

Pour des applications pharmaceutiques ou cosmétiques, les différents principes actifs et agents spécifiques peuvent être formulés avec 3,5 à 5% du polymère thermosensible, en solution aqueuse, à température ambiante. Les formulations liquides en dessous de 25°C peuvent être alors appliquées sous forme d'un spray sur la peau, sur les muqueuses vaginales ou nasales ; les formulations se gélifieront au contact. Les formulations crémeuses pourront être étalées sur la peau et s'épaissiront lors de l'arrêt de la friction. Dans ces deux exemples, la gélification de la formulation permettra une libération contrôlée et progressive des principes actifs.

L'augmentation de viscosité pourra également être mise à profit pour stabiliser la viscosité de crèmes solaires ou de peintures. En effet, si la chaleur fluidifie les produits cités, la présence du polymère thermosensible de l'invention dans les produits permet de compenser la diminution de viscosité par sa gélification (ou ici sa viscosification).

Ces polymères sont formulés en solution aqueuse pour former des compositions pharmaceutiques ou non pharmaceutiques, en particulier cosmétiques, susceptibles, notamment, d'être appliquées à basse viscosité sur le corps et de gélifier ensuite grâce à l'augmentation de la température.

Un premier exemple d'application concerne les préparations pharmaceutiques comportant un polymère thermosensible amélioré conforme à l'invention en solution dans l'eau et un principe actif. Ce type de préparation est appliquée sous forme sensiblement liquide sur le corps et elle gélifie ensuite de sorte que le principe actif est réparti sur toute la surface d'application et y est maintenu par le gel. Des collyres, par exemple, sont susceptibles d'être formulés ainsi. Selon une variante de mise en oeuvre ces préparations, contenant un principe actif, sont susceptibles d'être appliquées sur les muqueuses, notamment sur les muqueuses vaginales, nasales, celles de l'estomac ou de l'oesophage.

Un second exemple concerne les préparations destinées à être appliquées par voie sous-cutanée pour libérer un principe actif de façon lente ou pour combler des espaces ou des cavités, par exemple les rides. Des prothèses mammaires sont également susceptibles d'être constituées par un gel formulé avec un polymère thermosensible conforme à l'invention. Ainsi, la prothèse peut être introduite sous une forme liquide ce qui diminue la largeur de l'incision nécessaire.

Un autre exemple d'application concerne les bouchons méatiques, destinés à boucher le canal lacrymal. Le bouchon est réalisé en introduisant dans le fond de la cavité une solution contenant un polymère conforme à l'invention sous forme liquide à la température ambiante voisine de 20°C et en le laissant se réchauffer à la température du corps voisine de 37°C pour qu'il forme un bouchon dans le fond de la cavité.

Encore un autre exemple d'application concerne un vêtement ou un sous-vêtement comprenant au moins une partie contenant un gel thermoréversible obtenu à partir d'une solution aqueuse comportant un polymère thermosensible amélioré selon l'invention.

### EXEMPLES

### EXEMPLE 1:

Dans cet exemple, 3,8.10⁻³ mole d'un polymère portant la référence F127 commercialisé sous le nom de Pluronic est dissous dans 150 ml de 2-butanone, l'ensemble étant porté à 70°C. Le polymère F127 est introduit sans purification et contient 0,3% en masse d'eau (7,6.10⁻³ mole). Ensuite, 11,4. 10⁻³ mole de 4,4'-méthylène biscyclohexyl di-isocyanate correspondant à la formule générale : 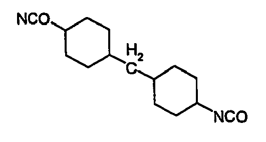 sont ajoutés goutte à goutte au mélange durant 10 minutes sous flux continu d'azote. Un catalyseur à base d'étain est introduit (à raison de 500 ppm) dans le mélange qui est maintenu au reflux du solvant pendant 20 heures environ.

Après que le mélange ait atteint la température ambiante, de l'ordre de 20°C, une quantité d'eau déterminée est ajoutée au mélange réactionnel de façon que le taux de polymère thermosensible ainsi synthétisé corresponde à 5% du poids total. La 2-butanone contenue dans le mélange est éliminée sous vide. La solution à 5% massique de polymère thermogélifiant amélioré ainsi obtenue est prête à l'usage.

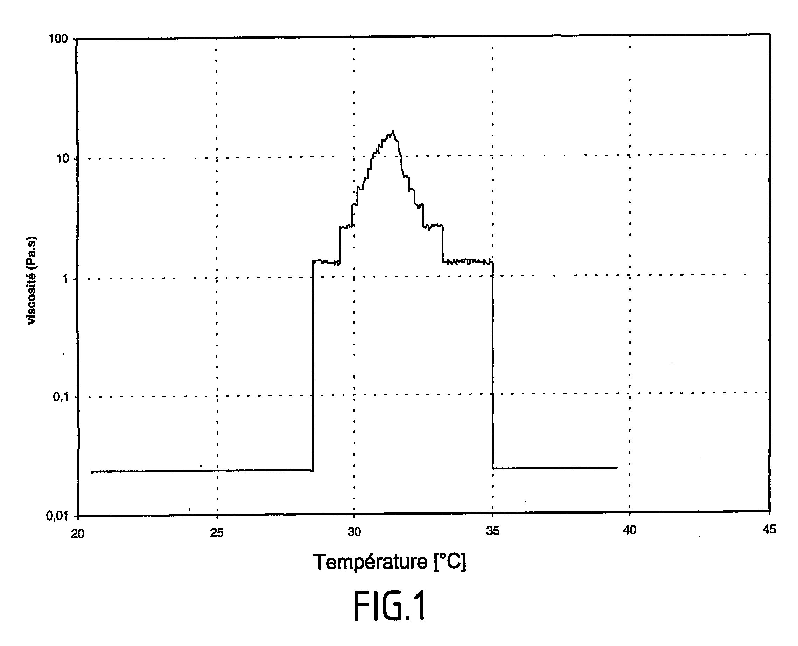

La Figure 1 illustre la courbe de viscosité du gel thermoréversible obtenu selon ce premier exemple, en fonction de la température sous une vitesse de cisaillement de 0,4/s. Ainsi, un avantage de ce premier exemple de mise en oeuvre de l'invention réside dans l'augmentation de la viscosité du gel d'une valeur supérieure à 10 Pa.s dans un intervalle de température compris entre 29 et 34°C avec une valeur maximale de la viscosité pour environ 32°C.

### EXEMPLE 2 :

Ce deuxième exemple de synthèse de polymère thermosensible amélioré correspondant au premier type de polymère décrit ci-dessus.

Seule la stoechiométrie de la réaction est sensiblement modifiée, la mise en oeuvre étant identique au premier exemple. Dans cet exemple, 8,0.10-³ mole de 4,4'-méthylène biscyclohexyl di-isocyanate sont introduit dans le mélange.

La solution de ce polymère amélioré est caractérisée par sa transparence et par une augmentation de 5 décades de la viscosité, sous une vitesse de cisaillement de 0,01 s⁻¹, lorsque sa température atteint 35°C.

### EXEMPLE 3

Cet exemple de synthèse de polymère thermosensible amélioré correspond au deuxième type de polymère décrit ci-dessus.

Le polymère thermosensible amélioré comprend des blocs polyacide et des blocs amino-tertiaires.

La synthèse s'effectue en deux étapes. Dans une première étape, 3,8.10⁻³ mole de polymère F127 Pluronic non séché contenant 0,3% en masse d'eau sont dissous avec 7,7.10⁻³ mole de N-méthyl diéthanolamine dans 150 ml de 2-butanone. Ensuite, 2,3.10⁻² mole de 4,4'-méthylène biscyclohexyl di-isocyanate sont ajoutés goutte à goutte durant 10 minutes sous flux continu d'azote. Puis, 1,2.10⁻² mole d'acide tartrique en solution dans la 2-butanone sont ajoutés après six heures de réaction à 70°C. La polycondensation se poursuit pendant 2 heures jusqu'à la disparition complète des fonctions isocyanate. Le polymère est recueilli soit directement en phase aqueuse, soit après transfert de solvant ou encore par précipitation dans l'éther ou l'hexane.

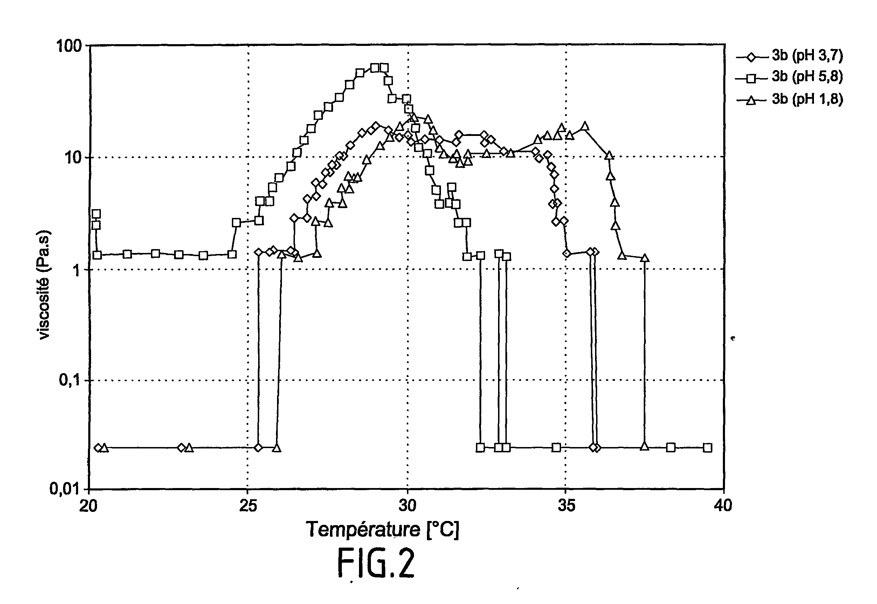

La Figure 2 illustre les variations de viscosité du gel obtenu avec 5% en poids du polymère thermosensible amélioré et synthétisé conformément à cet exemple. La Figure 2 présente 3 courbes correspondant à trois valeurs différentes du pH de la solution formant le gel 3,7 ; 5,8 ; et 1,8.

A la valeur de pH de 5,8, la viscosité du gel en fonction de la température se comporte de façon similaire au gel obtenu avec le polymère thermosensible amélioré du premier exemple, excepté que la viscosité maximale est plus importante et qu'elle atteint sensiblement 100 Pa.s à 29°C sous une vitesse de cisaillement de 0,4s⁻¹.

En revanche, à un pH de 3,7, la viscosité maximale du gel est moindre puisqu'elle est inférieure à 30 Pa.s, la courbe formant un plateau s'étendant sensiblement entre 28 et 34°C.

A un pH de 1,8, la courbe de viscosité a sensiblement la même forme décalée vers des températures plus élevées puisque le plateau s'étend entre 30 et 36°C.

Ainsi, grâce aux blocs ionisables du polymère thermosensible amélioré, il est possible d'ajuster la viscosité du gel en fonction de l'acidité de la solution dans laquelle on le dissous.

### EXEMPLE 4 :

Dans cet exemple de synthèse de polymère thermosensible amélioré correspondant au deuxième type de polymère décrit ci-dessus, on décrit un polymère thermosensible amélioré comprenant des blocs polyacides ionisables qui confèrent un caractère bioadhésif marqué au gel.

La synthèse s'effectue en deux étapes. Dans une première étape, 3,8.10⁻³ mole de polymère F127 Pluronic non séché contenant 0,3% en masse d'eau sont dissous dans 150 ml de 2-butanone. Ensuite, 1,5.10⁻² mole de 4,4'-méthylène biscyclohexyl di-isocyanate sont ajoutés goutte à goutte durant 10 minutes sous flux continu d'azote. Lorsque environ 81% des fonctions isocyanate ont été consommées, 2,9.10⁻³ mole d'acide 2,2-(bis hydroxymehtyl) butyrique sont ajoutés. La polycondensation se poursuit pendant 24 heures à 70°C jusqu'à la disparition complète des fonctions isocyanate. Le polymère est recueilli par précipitation dans l'éther diéthylique ; la viscosité dynamique d'une solution à 5% de ce polymère passe de 80mPa.s à température ambiante à 2400 Pa.s à 35°C, sous une vitesse de cisaillement de 0,003s⁻¹.

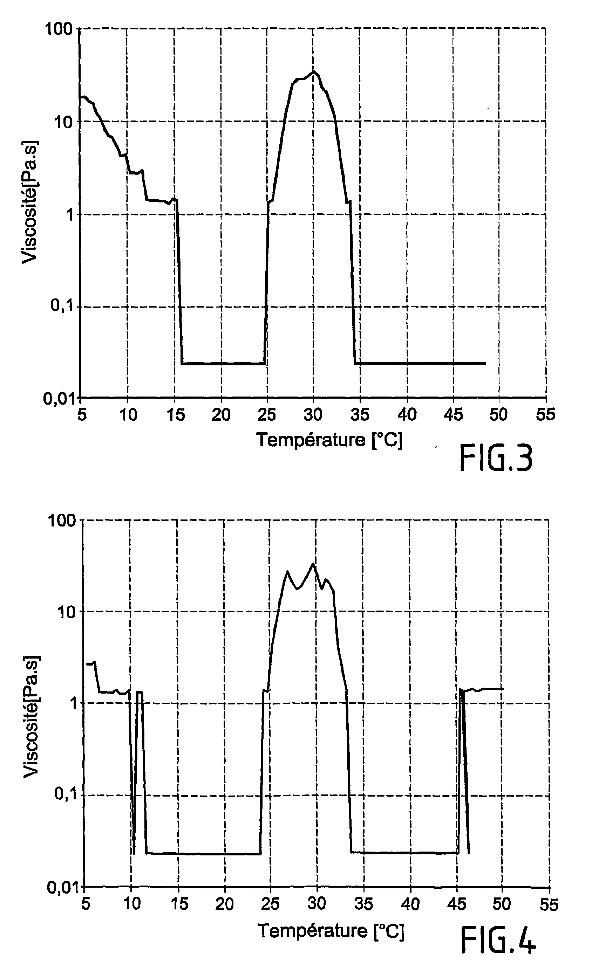

La Figure 3 donne les variations de viscosité du gel obtenu avec 5% en poids du polymère thermosensible synthétisé conformément à cet exemple, en fonction de la température sous une vitesse de cisaillement de 0,3/s.

### EXEMPLE 5 :

Dans cet exemple de synthèse, on réalise un polymère thermosensible amélioré présentant des ramifications; lesquelles portent des segments de polyoxyde d'éthylène de masse molaire de 750g.

La synthèse s'effectue en deux étapes ; une première étape consistant à réaliser un premier polymère conformément à la première étape l'exemple 4 et une seconde étape durant laquelle on introduit le polyoxyde d'éthylène monohydroxylé lorsqu'il ne reste plus que 26% des fonctions isocyanate initiales. La réaction se poursuit pendant 24 heures. Le gel formulé avec 5% de ce polymère présente une viscosité maximale à 37°C et il est très dense.

La Figure 4 donne la courbe de viscosité du gel thermoréversible obtenu selon cet exemple, en fonction de la température sous une vitesse de cisaillement de 0,3/s.

### EXEMPLE 6 :

Cet exemple de synthèse permet de produire un polymère thermosensible amélioré présentant des ramifications portant des amines aliphatiques à chaîne longue.

La synthèse comprend une première étape identique à la première étape de l'exemple précédent et une seconde étape dans laquelle on introduit de l'octadécyl amine dès qu'il ne reste plus que 18% des fonctions isocyanate initiales. Le gel formulé avec 8% dudit polymère présente une viscosité maximale à 37°C et il est très dense.

### EXEMPLE 7 :

3,8.10-³ mole d'un polymère portant la référence F127 commercialisé sous le nom de Pluronic est dissous dans 150 ml de 2-butanone, l'ensemble étant porté à 70°C. Le polymère F127 est introduit sans purification et contient 0,3% en masse d'eau (7,6.10-³ mole). Ensuite, 1,7. 10-² mole de 4,4'-méthylène biscyclohexyl di-isocyanate sont ajoutés goutte à goutte au mélange durant 10 minutes sous flux continu d'azote Un catalyseur à base d'étain est introduit 30 minutes après la fin de l'ajout des isocyanates, à raison de 2000 ppm dans le mélange qui est maintenu au reflux du solvant pendant 48 heures environ.

Lorsque la totalité des fonctions NCO a été consommée, le polymère est recueilli par précipitation dans l'éther diéthylique.

### EXEMPLE 8 :

Cet exemple de synthèse permet de produire un polymère thermosensible amélioré présentant une plus forte proportion de groupements urée ainsi que des ramifications.

La synthèse s'effectue en deux étapes ; une première étape consistant à réaliser un premier polymère par réaction de 3,8.10⁻³ mole de polymère F127 Pluronic non séché contenant 0,3% en masse d'eau (7,6.10⁻³ mole) et de 0,14g d'eau ajoutée (7,6.10⁻³ mole) avec 4,4.10⁻² mole de 4,4'-méthylène biscyclohexyl di-isocyanate ajouté goutte à goutte durant 10 minutes sous flux continu d'azote. Un catalyseur à base d'étain est introduit 30 minutes après la fin de l'ajout des isocyanates, à raison de 500 ppm dans le mélange qui est maintenu au reflux du solvant. Lorsque environ 81% des fonctions isocyanate ont été consommées, 2,9.10⁻³ mole d'acide 2,2-(bis hydroxymehtyl) butyrique sont ajoutées. La polycondensation se poursuit pendant 24 heures à 70°C jusqu'à la disparition complète des fonctions isocyanate initiales. Le polymère est recueilli par précipitation dans l'éther diéthylique. Le gel formulé avec 5% de ce polymère présente en solution une viscosité dynamique de 3000 Pa.s sous une vitesse de cisaillement de 0,003s⁻¹.

## Revendications

1. Polymère thermosensible hydrosoluble comprenant des chaînes linéaires de type polyloxyalkylène triblocs thermosensibles constituées de blocs de polyoxyde d'éthylène (POE) et de blocs polyoxyde de propylène (PPO), lesdites chaînes étant de la forme POE-PPO-POE, **caractérisé en ce que**
- ledit polymère consiste en une pluralité de chaînes linéaires de type polyoxyalkylène triblocs POE-PPO-POE thermosensible reliées entre elles,
- il comprend des enchaînements constitués d'au moins une chaîne POE-PPO-POE allongée à au moins une de ses extrémités par un groupement organique via une liaison carbamate et des enchaînements constitués d'au moins une chaîne POE-PPO-POE allongée à au moins une de ses extrémités par un groupement organique via une liaison urée ;
- ladite chaîne POE-PPO-POE répond à la formule 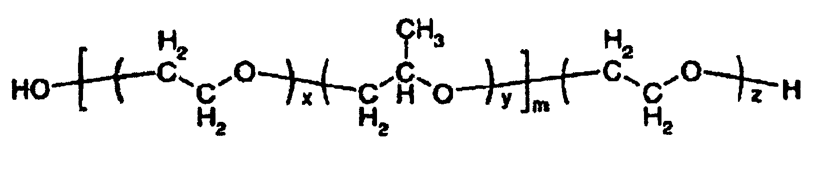 dans laquelle 20<x<120, 20<y<120, 20<z<120 et m>0,
- ledit polymère présente en solution dans l'eau à une concentration inférieure à 10% une faible viscosité à température ambiante et est apte à former des gels physiques à haut indice de viscosification à une température supérieure à 25°C.

2. Polymère thermosensible selon la revendication 1, **caractérisé en ce que** lesdits groupements organiques renferment des radicaux susceptibles d'être reliés auxdites chaînes via une liaison carbamate et sont choisis parmi :

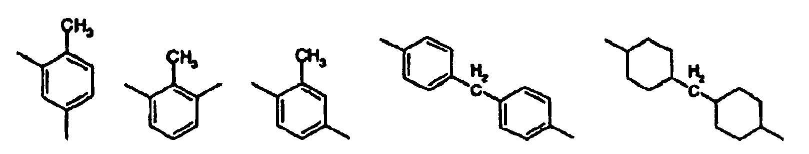

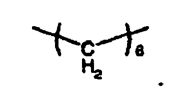

3. Polymère thermosensible selon l'une des revendications 1 ou 2, **caractérisé en ce que** lesdits groupements organiques renferment lesdits radicaux et des motifs acides reliés entre eux par des liaisons carbamate et/ou urée.

4. Polymère thermosensible selon l'une quelconque des revendications 1 à 3, **caractérisé en ce que** lesdits groupements organiques renferment lesdits radicaux et des motifs amine tertiaire reliés entre eux par des liaisons carbamate ou urée.

5. Polymère thermosensible selon l'une quelconque des revendications 1 à 4, **caractérisé en ce que** lesdits groupements organiques renferment une chaîne de type polyoxyde d'éthylène monohydroxylé.

6. Polymère thermosensible selon l'une quelconque des revendications 1 à 5, **caractérisé en ce que** lesdits groupements organiques présentent des ramifications constituées par des liaisons allophanate et/ou biuret.

7. Procédé de synthèse d'un polymère thermosensibte hydrosoluble, tel que défini dans l'une des revendications 1 à 6, ledit procédé étant **caractérisé en ce que** l'on fait réagir, en milieu solvant, au moins un polymère P linéaire de type polyoxyalkylène triblocs POE-PPO-POE thermosensible présentant au moins une fonction hydroxy terminale avec au moins une molécule organique porteuse d'au moins une fonction isocyanate, en présence d'eau dans le milieu réactionnel, de façon à les relier ensemble par des liaisons carbamates et urée.

8. Procédé selon la revendication 7 **caractérisé en ce qu'**il est mis en oeuvre en présence de 0,1 % à 0,6%, de préférence 0,3 à 0,6%, d'eau en masse par rapport au terpolymère.

9. Procédé selon la revendication 8, **caractérisé en ce que** de l'eau est ajoutée au milieu réactionnel à raison d'une quantité totale en eau de 0,1 % à 0,6%, de préférence 0,3 à 0,6%, massique par rapport au terpolymère.

10. Procédé selon l'une des revendications 7 ou 8, **caractérisé en ce que** ledit polymère P présente au moins deux fonctions hydroxy terminales.

11. Procédé selon l'une des revendications 9 ou 10, **caractérisé en ce que** ledit polymère P a pour formule générique: 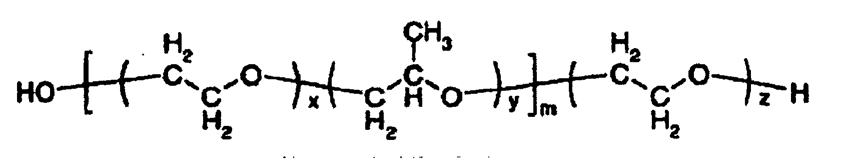 dans laquelle, 20< x <120, 20< y <120, 20< z <120, et m>0.

12. Procédé selon l'une quelconque des revendications 7 à 11, **caractérisé en ce que** la molécule organique comprend deux fonctions isocyanate.

13. Procédé selon .la revendication 11, **caractérisé en ce que** la molécule organique est choisie parmi :

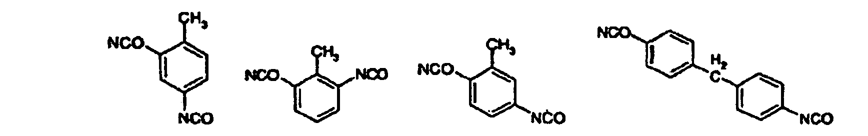

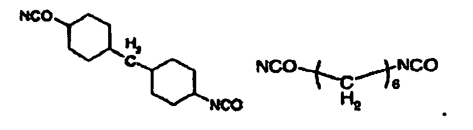

14. Procédé selon l'une des revendications 7 à 13, **caractérisé en ce que** l'on ajoute au moins une autre molécule organique porteuse d'au moins une fonction hydroxy, avantageusement deux, en présence d'eau.

15. Procédé selon la revendication 14, **caractérisé en ce que** ladite autre molécule organique comprend en outre au moins une fonction acide carboxylique, avantageusement deux.

16. Procédé selon la revendication 15, caractérisé en ce ladite autre molécule a pour formule :

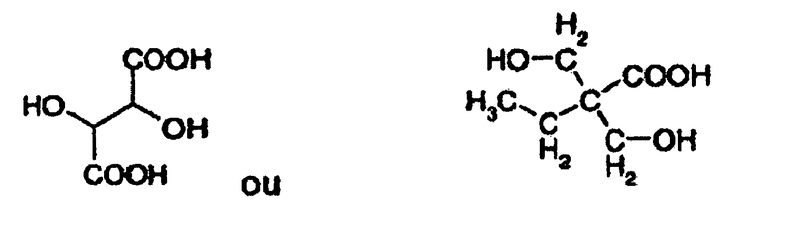

17. Procédé selon la revendication 14, **caractérisé en ce que** ladite autre molécule organique comprend au moins une fonction amine tertiaire.

18. Procédé selon la revendication 17, **caractérisé en ce que** ladite autre molécule a pour formule :

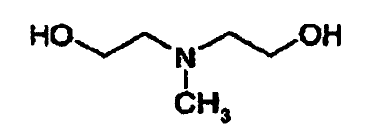

19. Procédé selon la revendication 14, **caractérisé en ce que** ladite autre molécule est un polyoxyde d'éthylène monohydroxylé.

20. Gel thermoréversible, **caractérisé en ce qu'**il est obtenu à partir d'une solution d'au moins un polymère selon l'une quelconque des revendications 1 à 6 ou d'au moins un polymère thermosensible obtenu par un procédé de synthèse selon l'une quelconque des revendications 7 à 19.

21. Gel thermoréversible selon la revendication 20, **caractérisé en ce qu'**il comprend de 1 à 10% en poids dudit polymère thermosensible, préférence de 1 à 5%.

22. Gel thermoréversible selon la revendication 20, **caractérisé en ce qu'**il contient de 0,05 à 0,1% en masse de polyacide carboxylique réticulé.

23. Gel thermoréversible selon la revendication 22, **caractérisé en ce qu'**il contient de 1 à 2,5% de polymère thermosensible.

24. Composition pharmaceutique ou non pharmaceutique, en particulier cosmétique, destinée au traitement du corps humain, **caractérisée en ce qu'**elle comprend un gel thermoréversible selon l'une des revendications 20 à 23.

25. Elément prothétique susceptible d'être inséré dans un organe du corps humain, **caractérisé en ce qu'**il comprend un gel thermoréversible selon l'une des revendications 20 à 23.

26. Vêtement ou sous-vêtement, **caractérisé en ce qu'**il comprend au moins une partie contenant un gel thermoréversible selon l'une des revendications 20 à 23.

## Claims

1. A hydrosoluble thermosensitive polymer which comprises linear chains of thermosensitive triblock polyoxyalkylene type constituted by blocks of poly(ethylene oxide) (PEO) and blocks of poly(propylene oxide) (PPO), said chains being in the form of PEO-PPO-PEO, **characterized in that**
- said polymer consists of a plurality of linear chains of thermosensitive PEO-PPO-PEO triblock polyoxyalkylene type bonded together,
- it comprises chain formations constituted by at least one PEO-PPO-PEO chain elongated at at least one of its termini by an organic group via a carbamate bond and chain formations constituted by at least one PEO-PPO-PEO chain elongated at at least one of its termini by an organic group via a urea bond;
- said PEO-PPO-PEO chain corresponding to formula 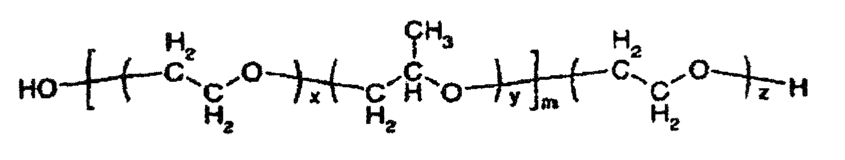 in which, 20<x<120, 20<y<120, 20<z<120, and m>0;
- said polymer has a low viscosity at ambient temperature in solution in water at a concentration of less than 10% and is capable of forming physical gels with a high viscosification index at a temperature greater than 25°C.

2. Thermosensitive polymer according to claim 1, **characterized in that** said organic groups contain radicals capable of being bonded to said chains via a carbamate bond and are chosen from:

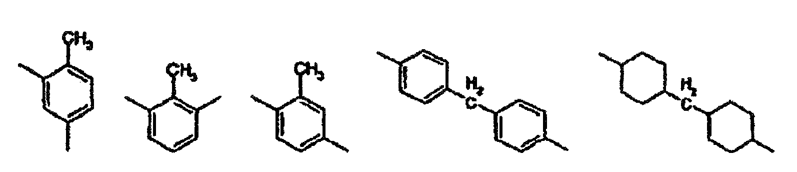

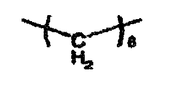

3. Thermosensitive polymer according to one of claims 1 or 2, **characterized in that** said organic groups contain said radicals and acid moieties bonded together by carbamate and/or urea bonds.

4. Thermosensitive polymer according to any one of claims 1 to 3, **characterized in that** said organic groups contain said radicals and tertiary amine moieties bonded together by carbamate or urea bonds.

5. Thermosensitive polymer according to any one of claims 1 to 4 **characterized in that** said organic groups contain a chain of monohydroxylated poly(ethylene oxide) type.

6. Thermosensitive polymer according to any one of claims 1 to 5 **characterized in that** said organic groups has branches constituted by allophanate and/or biuret bonds.

7. A method of synthesizing a hydrosoluble thermosensitive polymer as defined in one of claims 1 to 6, which method being **characterized in that** at least one linear polymer P of thermosensitive PEO-PPO-PEO polyoxyalkylene triblock type having at least one terminal hydroxy function is reacted in a solvent medium with at least one organic molecule which bears at least one isocyanate function, in the presence of water in the reaction medium, so as to bond them together with carbamate and urea bonds.

8. Method according to claim 7, **characterized in that** it is carried out in the presence of 0.1% to 0.6%, preferably 0.3 to 0.6%, of water by mass with respect to the terpolymer.

9. Method according to claim 8, **characterized in that** water is added to the reaction medium at the rate of a total quantity of water of 0.1 % to 0.6%, preferably 0.3 to 0.6%, by mass with respect to the terpolymer.

10. Method according to one of claims 7 or 8, **characterized in that** said polymer P has at least two terminal hydroxy functions.

11. Method according to one of claims 9 or 10 **characterized in that** said polymer P is of generic formula: 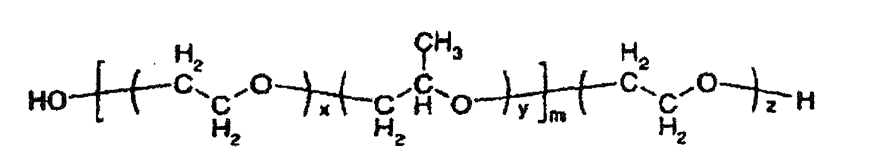 in which, 20<x<120, 20<y<120, 20<z<120, and m>0;

12. Method according to any one of claims 7 to 11, **characterized in that** the organic molecule comprises two isocyanate functions.

13. Method according to any claim 11, **characterized in that** the other organic molecule is selected from:

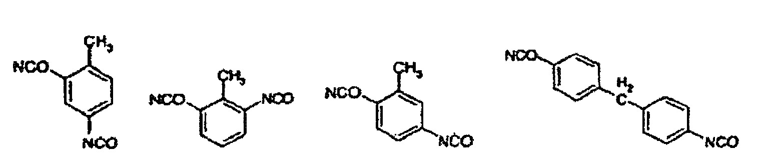

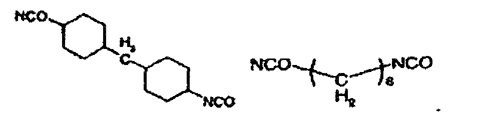

14. Method according to any one of claims 7 to 13, **characterized in that** at least one other organic molecule which bears at least one hydroxy function, advantageously two, is/are added in the presence of water.

15. Method according to claim 14 **characterized in that** said other organic molecule further comprises at least one carboxylic acid function, advantageously two.

16. Method according to claim 15 **characterized in that** said other molecule is of formula:

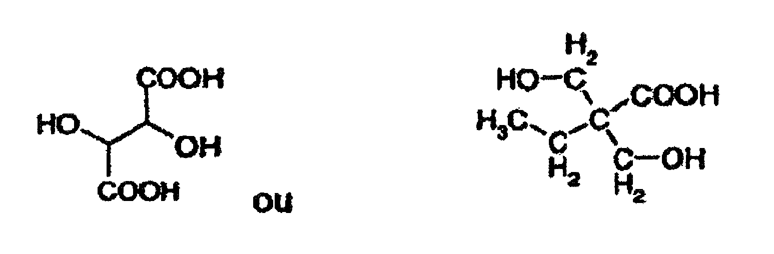

17. Method according to claim 14 **characterized in that** said other organic molecule comprises at least one tertiary amine function.

18. Method according to claim 17 **characterized in that** said other molecule is of formula:

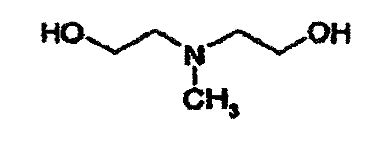

19. Method according to claim 14 **characterized in that** said other molecule is a monohydroxylated poly(ethylene oxide).

20. Thermoreversible gel **characterized in that** it is obtained from a solution of at least one thermosensitive polymer according to any one of claims 1 to 6 or at least one thermosensitive polymer obtained by a synthesis method according to any one of claims 7 to 19.

21. Thermoreversible gel according to claim 20 **characterized in that** it comprises 1 to 10% by weight of said thermosensitive polymer, preferably 1 to 5%.

22. Thermoreversible gel according to claim 20 **characterized in that** it contains 0.05 to 0.1% by weight of cross-linked poly(carboxylic acid).

23. Thermoreversible gel according to claim 22 **characterized in that** it contains 1 to 2.5% of thermosensitive polymer.

24. Pharmaceutical or non pharmaceutical, in particular cosmetic, composition intended for the treatment of the human body, **characterized in that** it comprises a thermoreversible gel according to one of claims 20 to 23.

25. Prosthetic element capable of being inserted into an organ of the human body, **characterized in that** it comprises a thermoreversible gel according to one of claims 20 to 23.

26. Garment or under-garment, **characterized in that** it comprises at least one part containing a thermoreversible gel according to one of claims 20 to 23.

## Patentansprüche

1. Wasserlösliches, thermosensibles Polymer mit linearen Ketten vom Typ thermosensibler Polyoxyalkylen-Triblocks, die aus Polyoxyethylen-Blöcken (POE) und aus Polyoxypropylen-Blöcken (PPO) bestehen, wobei die Ketten die Form POE-PPO-POE haben,
**dadurch gekennzeichnet , dass**
- das Polymer aus einer Mehrzahl von linearen Ketten vom Typ thermosensibler POE-PPO-POE-Polyoxyalkylen-Triblocks besteht, die miteinander verbunden sind,
- es Kettenbildungen umfasst, die aus zumindest einer POE-PPO-POE-Kette bestehen, die an zumindest einem ihrer Enden über eine Carbamat-Bindung mit einer organischen Gruppe verlängert ist, sowie Kettenbildungen, die aus zumindest einer POE-PPO-POE-Kette bestehen, die an zumindest einem ihrer Enden über eine Harnstoff-Bindung mit einer organischen Gruppe verlängert ist,
- wobei die POE-PPO-POE-Kette der nachfolgenden Formel entspricht: 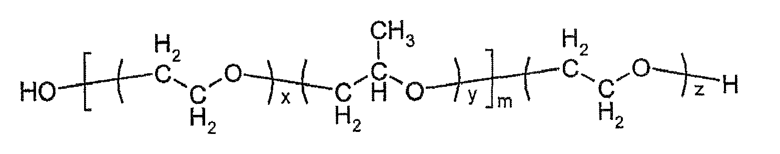 worin 20 < x < 120, 20 < y < 120, 20 < z < 120 und m > 0 ist,
- das Polymer in wässriger Lösung mit einer Konzentration von unter 10 % eine geringe Viskosität bei Raumtemperatur aufweist und dazu geeignet ist, physikalische Gele mit hohem Viskositätsindex bei einer Temperatur von über 25 °C zu bilden.

2. Thermosensibles Polymer nach Anspruch 1,
**dadurch gekennzeichnet , dass**
die organischen Gruppen Radikale einschließen, die sich über eine Carbamat-Bindung mit den genannten Ketten verbinden können und ausgewählt sind aus:

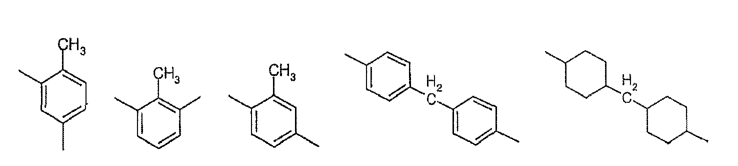

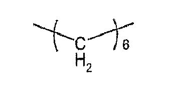

3. Thermosensibles Polymer nach einem der Ansprüche 1 oder 2,
**dadurch gekennzeichnet , dass**
die organischen Gruppen die genannten Radikale und Säureeinheiten einschließen, die über Carbamat- und/oder Harnstoff-Bindungen miteinander verbunden sind.

4. Thermosensibles Polymer nach einem der Ansprüche 1 bis 3,
**dadurch gekennzeichnet , dass**
die organischen Gruppen die genannten Radikale und Einheiten tertiärer Amine einschließen, die über Carbamat- oder Harnstoff-Bindungen miteinander verbunden sind.

5. Thermosensibles Polymer nach einem der Ansprüche 1 bis 4,
**dadurch gekennzeichnet , dass**
die organischen Gruppen eine Kette vom Typ Monohydroxy-Polyoxymethylen einschließen.

6. Thermosensibles Polymer nach einem der Ansprüche 1 bis 5,
**dadurch gekennzeichnet , dass**
die organischen Gruppen Verzweigungen aufweisen, die aus Allophan- und/oder Biruet-Bindungen bestehen.

7. Verfahren zur Synthese eines wasserlöslichen, thermosensiblen Polymers wie in einem der Ansprüche 1 bis 6 definiert, wobei das Verfahren
**dadurch gekennzeichnet ist, dass**
man in einem Lösungsmittelmedium zumindest ein lineares Polymer P vom Typ thermosensibler POE-PPO-POE Polyoxyalkylen-Triblock-Polymere, das zumindest eine terminale Hydroxy-Funktion aufweist, mit zumindest einem organischen Molekül als Träger zumindest einer Isocyanat-Funktion reagieren lässt, und zwar in Gegenwart von Wasser in dem Reaktionsmedium, so dass sie durch Carbamat- und Harnstoff-Bindungen miteinander verbunden werden.

8. Verfahren nach Anspruch 7,
**dadurch gekennzeichnet , dass**
es in Gegenwart von 0,1 Masse% bis 0,6 Masse%, vorzugsweise 0,3 Masse% bis 0,6 Masse%, Wasser bezüglich des Terpolymers durchgeführt wird.

9. Verfahren nach Anspruch 8,
**dadurch gekennzeichnet , dass** dem Reaktionsmedium Wasser in einer Wassergesamtmenge von 0,1 Masse% bis 0,6 Masse%, vorzugsweise 0,3 bis 0,6 Masse%, bezüglich des Terpolymers hinzugefügt wird.

10. Verfahren nach einem der Ansprüche 7 oder 8,
**dadurch gekennzeichnet , dass**
das Polymer P zumindest zwei terminale Hydroxy-Funktionen aufweist.

11. Verfahren nach einem der Ansprüche 9 oder 10,
**dadurch gekennzeichnet , dass**
das Polymer P folgende generische Formel hat: 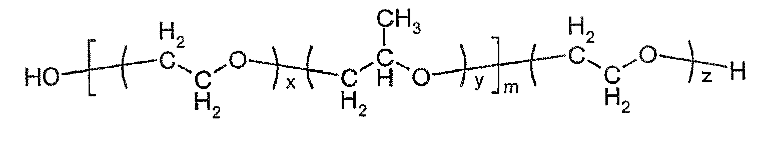 worin 20 < x < 120, 20 < y < 120, 20 < z < 120 und m > 0 ist.

12. Verfahren nach einem der Ansprüche 7 bis 11,
**dadurch gekennzeichnet , dass**
das organische Molekül zwei Isocyanat-Funktionen aufweist.

13. Verfahren nach Anspruch 11,
**dadurch gekennzeichnet , dass**
das organische Molekül ausgewählt ist aus:

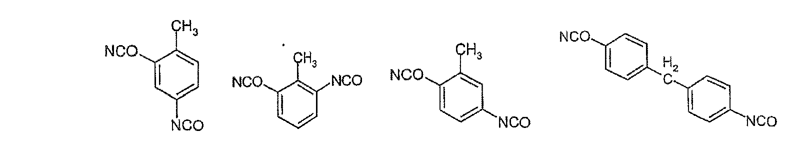

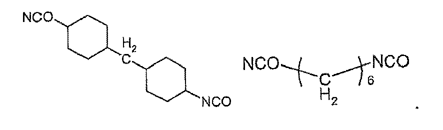

14. Verfahren nach einem der Ansprüche 7 bis 13,
**dadurch gekennzeichnet , dass**
zumindest ein weiteres Molekül als Träger zumindest einer, vorzugsweise zweier Hydroxy-Funktion(en) in Gegenwart von Wasser zugefügt wird.

15. Verfahren nach Anspruch 14,
**dadurch gekennzeichnet , dass**
das weitere organische Molekül ferner zumindest eine, vorzugsweise zwei Carbonsäure-Funktion(en) aufweist.

16. Verfahren nach Anspruch 15,
**dadurch gekennzeichnet , dass**
das weitere Molekül als Formel hat:

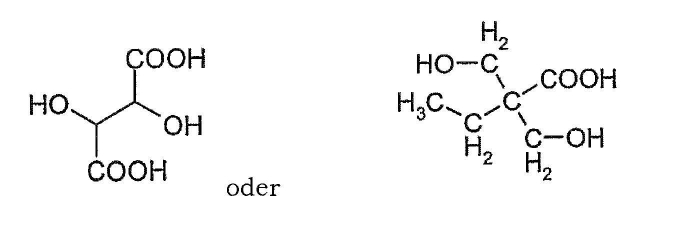

17. Verfahren nach Anspruch 14,
**dadurch gekennzeichnet , dass**
das weitere organische Molekül zumindest eine tertiäre AminFunktion aufweist.

18. Verfahren nach Anspruch 17,
**dadurch gekennzeichnet , dass**
das weitere Molekül als Formel hat:

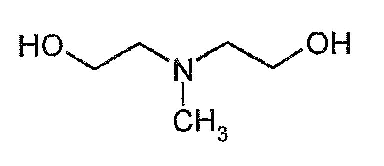

19. Verfahren nach Anspruch 14,
**dadurch gekennzeichnet , dass**
das weitere Molekül ein Monohydroxy-Polyoxyethylen ist.

20. Thermoreversibles Gel,
**dadurch gekennzeichnet , dass**
es ausgehend von einer Lösung aus zumindest einem Polymer nach einem der Ansprüche 1 bis 6 oder aus zumindest einem thermosensiblen Polymer erhalten wird, das mit einem Syntheseverfahren nach einem der Ansprüche 7 bis 19 erhalten wird.

21. Thermoreversibles Gel nach Anspruch 20,
**dadurch gekennzeichnet , dass**
es 1 bis 10 Gew.-%, vorzugsweise 1 bis 5 Gew.-%, thermosensibles Polymer enthält.

22. Thermoreversibles Gel nach Anspruch 20,
**dadurch gekennzeichnet , dass**
es 0,05 bis 0,1 Masse% vernetzte Polycarbonsäure enthält.

23. Thermoreversibles Gel nach Anspruch 22,
**dadurch gekennzeichnet , dass**
es 1 bis 2,5 % thermosensibles Polymer enthält.

24. Pharmazeutische oder nicht pharmazeutische, insbesondere kosmetische Zusammensetzung, die zur Behandlung des menschlichen Körpers bestimmt ist,
**dadurch gekennzeichnet , dass**
sie ein thermoreversibles Gel nach einem der Ansprüche 20 bis 23 enthält.

25. Prothese-Element, das dazu geeignet ist, in ein Organ des menschlichen Körpers eingesetzt zu werden,
**dadurch gekennzeichnet , dass**
es ein thermoreversibles Gel nach einem der Ansprüche 20 bis 23 enthält.

26. Kleidung oder Unterkleidung,
**dadurch gekennzeichnet , dass**
sie zumindest einen Bereich aufweist, der ein thermoreversibles Gel nach einem der Ansprüche 20 bis 23 beinhaltet.
